# EUROPEAN PATENT SPECIFICATION

(11) **EP 4 268 743 B1**
(45) Date of publication and mention of the grant of the patent: **07.08.2024**
(21) Application number: 23197823.0
(22) Date of filing: 30.09.2021
(51) Int. Cl.: A61N 1/40, A61N 1/06, A61N 1/36, A61B 18/00

(54) **IMPLANTABLE ARRAYS FOR PROVIDING TUMOR TREATING FIELDS**
IMPLANTIERBARE ARRAYS ZUR BEREITSTELLUNG VON TUMORBEHANDLUNGSFELDERN
RÉSEAUX IMPLANTABLES POUR FOURNIR DES CHAMPS DE TRAITEMENT DE TUMEUR

(30) Priority: 30.09.2020 US 202063085603 P
(43) Date of publication of application: 01.11.2023
(62) Divisional of application: 21789862.6
(73) Proprietor: Novocure GmbH, 6039 Root D4 (CH)
(72) Inventor: CARLSON, Kristen, Concord, 01742 (US)
(74) Representative: Boden, Keith McMurray

(56) References cited:
- WO-A1-2020/174429
- US-A1- 2015 066 024
- US-A1- 2018 126 151
- US-B1- 6 692 490

## Description

### FIELD

This application relates generally to apparatuses for providing tumor treating fields and, in particular, for apparatuses for implanting electrodes within a patient for providing tumor treating fields.

### BACKGROUND

Tumor Treating Fields, or TTFields, are low intensity (e.g., 1-6 V/cm) alternating electrical fields within an intermediate frequency range (e.g. 50-500 kHz). This heretofore non-invasive treatment targets solid tumors and is described in U.S. Pat. No. 7,565,205, which is incorporated herein by reference in its entirety. TTFields disrupt cell division through physical interactions with key molecules during mitosis. TTFields therapy is an approved mono-treatment for recurrent glioblastoma, and an approved combination therapy with chemotherapy for newly diagnosed patients. Conventionally, these electrical fields are induced non-invasively by transducer arrays (i.e., arrays of electrodes) placed directly on the patient's scalp. TTFields also appear to be beneficial for treating numerous tumor cell types in many other parts of the body.

WO-A-2020/174429 discloses an apparatus for delivering tumor treating fields, which comprises a plurality of sets of electrode elements, each of the sets of electrode elements being configured for implantation within a person's body, and a plurality of temperature sensors configured for implantation within the person's body and positioned with respect to the sets of electrode elements to measure a temperature at each of the sets of electrode elements.

US-A-6692490 discloses a method and system for treating disorders of the genitourinary tract and other disorders in other parts of the body, including one or more of, or some combination of, ablation, nerve modulation, three-dimensional tissue shaping, drug delivery, mapping, stimulating, shrinking (by creation of a pattern of thermal lesions) and reducing strain on structures by altering the geometry thereof and providing bulk to particularly defined regions.

US-A-2015/0066024 discloses devices and systems, as well as methods, of electric field delivery and non-thermal or selective ablation of target tissue regions, including selective ablation of cancerous cells and solid tumors, which includes delivering an electric field to a tissue, including positioning an electrode within a target tissue region comprising cancerous cells, and applying an alternating electrical current to the target tissue so as non-thermally to ablate cancerous cells of the target tissue region around the electrodes.

US-A-2018/0126151 discloses electrode systems for transvascular stimulation of target nerves, which include electrode arrays, elements promoting blood flow between electrode surfaces and surrounding vascular walls, electrodes shaped to promote more even current density than electrodes having angular edges, features for retaining the electrode systems with blood vessels, and features for using electrode-carrying elements to asymmetrically distend blood vessel walls towards target nerve structures.

### SUMMARY

Described herein, in various aspects, is an implantable apparatus for providing tumor treating fields (TTFields) according to claim 1.

Additional advantages of the invention will be set forth in part in the description that follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE DRAWINGS

These and other features of the preferred embodiments of the invention will become more apparent in the detailed description in which reference is made to the appended drawings wherein:
FIG. 1 is a block diagram of a system for delivering tumor treating fields using implantable apparatuses and systems as disclosed herein.
FIG. 2 is side view of an implantable apparatus for providing TTFields in accordance with embodiments disclosed herein.
FIG. 3 is a schematic diagram of an electrode array of an implantable apparatus as disclosed herein.
FIG. 4 is a plurality of schematic diagrams of an electrode array at various time intervals, showing respective polarities of each electrode of the electrode array, in accordance with one aspect.
FIG. 5 is a plurality of schematic diagrams of an electrode array at various time intervals, showing respective polarities of each electrode of the electrode array, in accordance with another aspect.
FIG. 6A illustrates a schematic diagram of a first stimulating electrode array, showing respective polarities of each electrode of the first stimulating electrode array; and FIG. 6B illustrates a schematic diagram of a second stimulating electrode array, showing respective polarities of each electrode of the second stimulating electrode array in accordance with one aspect.
FIG. 7A illustrates a schematic diagram of a first stimulating electrode array, showing respective polarities of each electrode of the first stimulating electrode array; and FIG. 7B illustrates a schematic diagram of a second stimulating electrode array, showing respective polarities of each electrode of the second stimulating electrode array, in accordance with another aspect.

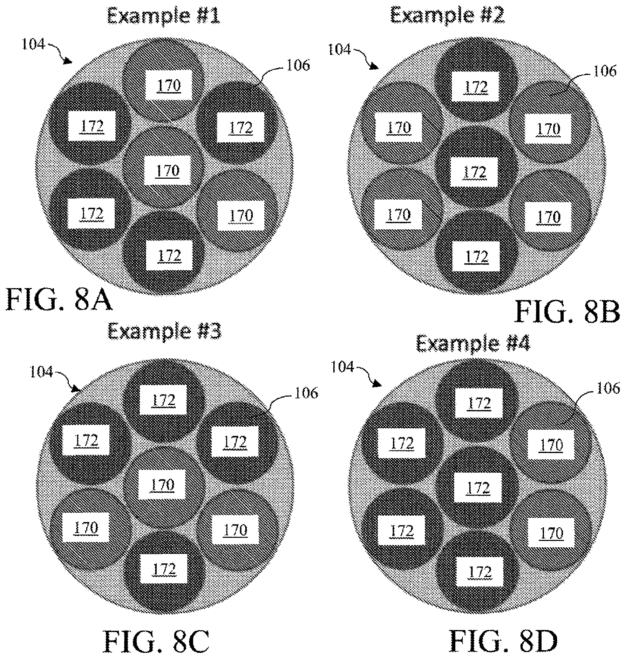

FIG. 8A illustrates a schematic diagram of a stimulating electrode array, showing respective polarities of each electrode of each array, in accordance with at least one aspect. FIG. 8B illustrates a schematic diagram of a stimulating electrode array, showing respective polarities of each electrode of each array, in accordance with another aspect. FIG. 8C illustrates a schematic diagram of a stimulating electrode array, showing respective polarities of each electrode of each array, in accordance with yet another aspect. FIG. 8D illustrates a schematic diagram of a stimulating electrode array, showing respective polarities of each electrode of each array, in accordance with still another aspect.
FIG. 9 illustrates a model of TTFields propagating through tissue as generated by an implantable apparatus as disclosed herein.
FIG. 10 illustrates a model of TTFields propagating through tissue as generated by an implantable apparatus as disclosed herein.
FIG. 11 illustrates a model of TTFields propagating through tissue as generated by an implantable apparatus as disclosed herein.
FIG. 12 is a schematic diagram of a patient having an implantable apparatus disposed within her skull, a transcranial stimulation device outside the skull, with the target site positioned between the implantable apparatus and the transcranial stimulation device.

### DETAILED DESCRIPTION

The disclosed system and method may be understood more readily by reference to the following detailed description of particular embodiments and the examples included therein and to the Figures and their previous and following description.

It is to be understood that the terminology used herein is for the purpose of describing particular embodiments only and is not intended to limit the scope of the present invention which will be limited only by the appended claims.

It must be noted that as used herein and in the appended claims, the singular forms "a," "an," and "the" include plural references unless the context clearly dictates otherwise. Thus, for example, reference to "an electrode" includes one or more of such electrodes, and so forth.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Optionally, in some aspects, when values are approximated by use of the antecedents "about," "substantially," "approximately," or "generally," it is contemplated that values within up to 15%, up to 10%, up to 5%, or up to 1% (above or below) of the particularly stated value or characteristic can be included within the scope of those aspects.

Unless defined otherwise, all technical and scientific terms used herein have the same meanings as commonly understood by one of skill in the art to which the disclosed apparatus, system, and method belong.

As used herein, the term "patient" refers to a human or animal subject who is in need of treatment using the disclosed systems and devices.

As used herein, the term "electrode" refers to any structure that permits generation of an electric potential, electric current, or electrical field as further disclosed herein. Optionally, an electrode can comprise a transducer. Optionally, an electrode can comprise a non-insulated portion of a conductive element.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of'), meaning that each step is not intended to exclude, for example, other additives, components, integers or steps that are not listed in the step.

Unless otherwise expressly stated, it is in no way intended that any method or aspect set forth herein be construed as requiring that its steps be performed in a specific order. Accordingly, where a method claim does not specifically state in the claims or description that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of embodiments described in the specification.

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any manner. Embodiments illustrated under any heading or in any portion of the disclosure may be combined with embodiments illustrated under the same or any other heading or other portion of the disclosure.

Any combination of the elements described herein in all possible variations thereof is encompassed by the invention unless otherwise indicated herein or otherwise clearly contradicted by context.

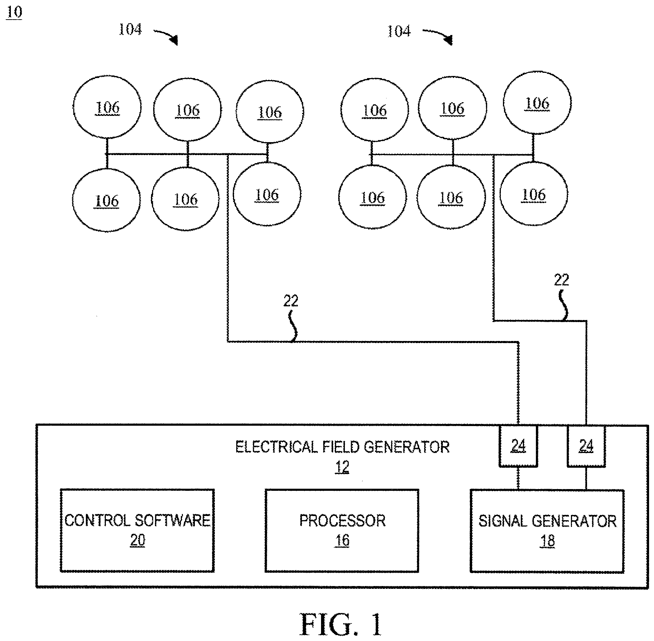

FIG. 1 shows an example apparatus 10 for electrotherapeutic treatment. Generally, the apparatus 10 may be a portable battery or power supply operated device that produces alternating electrical fields within the body by means of transducer arrays or other electrodes. The apparatus 10 may comprise an electrical field generator 12 and one or more electrode (e.g., transducer) arrays 104, each comprising a plurality of electrodes 106. The apparatus 10 may be configured to generate tumor treating fields (TTFields) (e.g., at 150 kHz for one tumor cell type, or 300 kHz for a different tumor cell type) via the electrical field generator 12 and deliver the TTFields to an area of the body through the one or more electrode arrays 104. The electrical field generator 12 may be a battery and/or power supply operated device.

The electrical field generator 12 may comprise a processor 16 in communication with a signal generator 18. The electrical field generator 12 may comprise control software 20 configured for controlling the performance of the processor 16 and the signal generator 18.

The signal generator 18 may generate one or more electric signals in the shape of waveforms or trains of pulses. The signal generator 18 may be configured to generate an alternating voltage waveform at frequencies in the range from about 50 kHz to about 500 kHz (preferably from about 100 kHz to about 300 kHz) (e.g., the TTFields). The voltages are such that the electrical field intensity in tissue to be treated is typically in the range of about 0.1 V/cm to about 10 V/cm.

One or more outputs 24 of the electrical field generator 12 may be coupled to one or more conductive leads 22 that are attached at one end thereof to the signal generator 18. The opposite ends of the conductive leads 22 are connected to the one or more electrode arrays 104 that are activated by the electric signals (e.g., waveforms). The conductive leads 22 may comprise standard isolated conductors with a flexible metal shield and may be grounded to prevent the spread of the electrical field generated by the conductive leads 22. The one or more outputs 24 may be operated sequentially. Output parameters of the signal generator 18 may comprise, for example, an intensity of the field, a frequency of the waves (e.g., treatment frequency), and a maximum allowable temperature of the one or more electrode arrays 104. The output parameters may be set and/or determined by the control software 20 in conjunction with the processor 16. After determining a desired (e.g., optimal) treatment frequency, the control software 20 may cause the processor 16 to send a control signal to the signal generator 18 that causes the signal generator 18 to output the desired treatment frequency to the one or more electrode arrays 104. It is further contemplated that the control software 20 can cause the processor 1 6 to shift or change the direction of TTFields or otherwise adjust the properties of TTFields in the manner further disclosed herein.

The one or more electrode arrays 104 may be configured in a variety of shapes and positions so as to generate an electrical field of the desired configuration, direction and intensity at a target volume so as to focus treatment. Optionally, the one or more electrode arrays 104 may be configured to deliver two electric fields in two different directions, or even three electric fields in three different directions, through the volume of interest. Optionally, the one or more electrode arrays 104 may be configured to deliver two perpendicular field directions, or even three orthogonal field directions, through the volume of interest.

Further disclosure directed to use of such electrotherapeutic systems is provided in U.S. Patent Application Publication No. 2021/0162228 to Urman et al., published June 3, 2021.

Although transducers are conventionally positioned externally on the patient, the present disclosure recognizes that there are benefits to positioning electrodes within the body of the patient to provide localized electric fields at the site of the tumor.

In use, it is contemplated that changing the direction at which the electric field generated by TTFields is oriented with regard to cellular membranes and molecules can provide improved tumor-killing efficacy. As more changes of direction are applied, the variance of field strength as seen by the target structures will decrease, and fewer structures will be subjected to weak, sub-efficacy threshold field strength.

TTFields are FDA-approved for treating brain cancer. (e.g., glioblastoma). However, delivering TTFields to target regions in the brain via trans-dermal arrays can be particularly challenging, primarily due to high resistivity of the skull. Further, preferred locations for the arrays on the skull may be partially obstructed by the ears. The present invention provides a location for one or more array within the skull, negating the issue of high resistivity of the skull as well as avoiding obstructions on the skin surface. Because resection (removal) of the tumor often occurs prior to treatment with TTFields, the current apparatus can be inserted into the void space left from resection, and in some embodiments, the apparatus may be inflated to fill the void space. The latter approach has the additional benefit of placing the one or more array in close proximity to stray cancer cells or clusters that may have been missed during resection, which advantage extends to cancers in other parts of the human (mammalian) body.

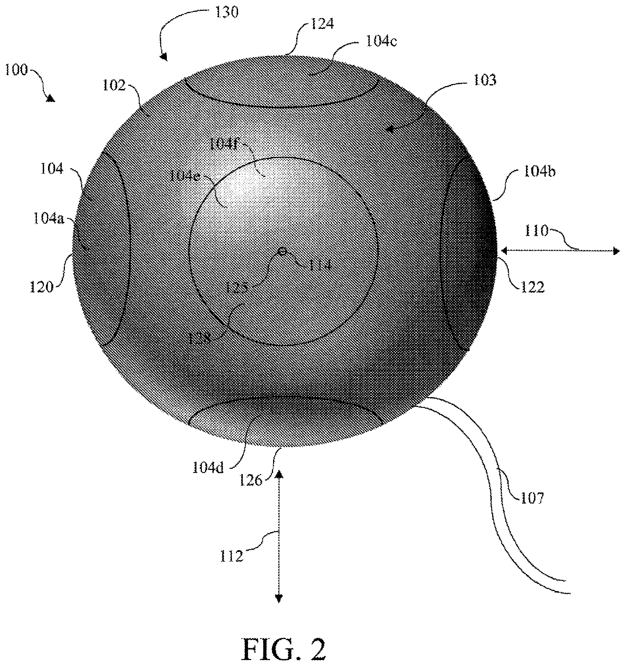

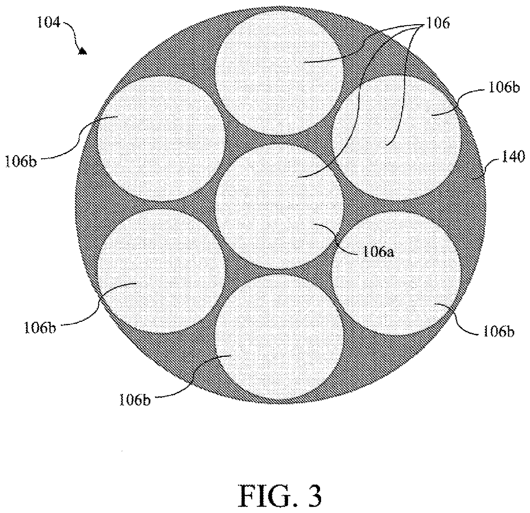

Disclosed herein, in various aspects and with reference to FIGS. 2 and 3, is an implantable apparatus 100 for providing TTFields. The implantable apparatus 100 can comprise a support structure 102 (e.g., a body or primary structure of the apparatus) and at least one electrode array (optionally, a plurality of electrode arrays) 104 disposed on (and supported by) the support structure. Each electrode array can comprise a plurality of electrodes 106. The electrode arrays 104 can be distributed evenly (e.g., equally spaced) around the support structure or in an uneven distribution, depending on the desired application and effect.

The support structure 102 can optionally be inflatable from a collapsed configuration to an expanded configuration. Thus, it is contemplated that the support structure 102 can function as a balloon that can be selectively inflated or deflated to adjust positioning of electrode arrays 104 within or relative to the body of a subject. For example, the support structure 102 can define an interior 103 that can be in fluid communication with a conduit 107. The conduit 107 can be configured to receive fluid therethrough for inflating the support structure 102. Embodiments of said fluid may be desirable for the fluid's biological properties, such as being anti-bacterial, tumoricidal, biologically compatible, or inert. Embodiments of the fluid can also be desirable for the fluid's thermodynamic properties, such as heat carrying or heat absorbing capacity. Embodiments of the fluid can also be desirable for the fluid's electrical properties, such as ability to reflect, shield, transmit, or guide an electric field, which can allow a stronger and more efficacious electric field to be generated in the tumor vicinity. The fluid may be conductive, or it may be non-conductive. Accordingly, the fluid can be selected based on its desired biological properties, thermodynamic properties, and/or electrical properties. In this way, the support structure 102 can be configured to be inflated from a collapsed configuration to an expanded configuration. In some aspects, the support structure 102, when in the expanded configuration, can be spherical, ovoid, cylindrical, oblong, elongate, flat, curved, amorphous, or any suitable shape. The support structure 102 can optionally comprise at least one flexible wall that can be configured to expand from fluid pressure within the interior 103 of the support structure 102.

In exemplary aspects, and with reference to FIG. 2, the at least one electrode array can comprise a plurality of electrode arrays 104. In some optional aspects, the implantable apparatus 100 can comprise a first electrode array 104a disposed on a first portion 120 of the support structure 102 and a second electrode array 104b disposed on a second portion 122 of the support structure. Optionally, the first and second portions 120, 122 of the support structure 102 can be centered or generally centered on opposing sides of the support structure. In optional aspects, the implantable apparatus 100 can further comprise a third electrode array 104c disposed on a third portion 124 of the support structure 102 and a fourth electrode array 104d disposed on a fourth portion 126 of the support structure. Optionally, the third and fourth portions 124, 126 of the support structure 102 can be centered or generally centered on opposing sides of the support structure. In still further optional aspects, the implantable apparatus 100 can comprise a fifth electrode array 104e disposed on a fifth portion 128 of the support structure 102 and a sixth electrode array 104f disposed on a sixth portion (not shown) of the support structure. Optionally, the fifth portion 128 and the sixth portion of the support structure 102 can be centered or generally centered on opposing sides of the support structure. Accordingly, optionally, in some aspects, the implantable apparatus 100 can comprise six electrode arrays 104 spaced about the support structure 102. However, it is contemplated that any desired number of electrode arrays, including 1, 2, 3, 4, 5, 7, 8, 9, 10, 11, 12, 13, 14, 15, or 16 electrode arrays, can be associated with the support structure. It will be understood by those skilled in the art that the exemplary electrode arrays disclosed herein can advantageously maximize the number of electric field patterns that can be applied to kill tumor cells in a target region. However, it is contemplated that the exemplary arrays should be understood to be non-limiting examples, and the scope of this disclosure and claims are not limited to the examples given herein. Further, those skilled in the art will understand that some electrode configurations and designs are limited by current manufacturing technology, but different manufacturing technology in the future will permit different electrode configurations.

In exemplary aspects, and as further described above, the plurality of electrode arrays 104 can comprise a first electrode array 104a disposed on the first portion 120 of the support structure 102 and a second electrode array 104b disposed on the second portion 122 of the support structure. In these aspects, in the expanded configuration, the first electrode array 104a and the second electrode array 104b can be aligned in a first direction. In exemplary aspects, the first direction can be coincident with a first axis 110 that extends between respective centroids 125 of the first and second electrode arrays 104a, 104b. As used herein, the term "centroid" can refer to the geometric center of the area occupied by a given object or structure.

In further optional aspects, and as further described above, the plurality of electrode arrays 104 can further comprise a third electrode array 104c disposed on the third portion 124 of the support structure 102 and a fourth electrode array 104d disposed on the fourth portion 126 of the support structure. In the expanded configuration, the third electrode array 104c and the fourth electrode array 104d can be aligned in a second direction that is different than the first direction. In exemplary aspects, the second direction can be coincident with a second axis 112 that extends between respective centroids 125 of the third and fourth electrode arrays 104c, 104d. Optionally, in these aspects, it is contemplated that the first and second axes 110, 112 do not intersect. In further aspects, it is contemplated that the first and second axes 110, 112 can intersect. Optionally, in these aspects, it is contemplated that the second axis 112 can be orthogonal (e.g., perpendicular) or within 1 degree, 5 degrees, 10 degrees, or 15 degrees of being orthogonal or perpendicular to the first axis 110.

In still further optional aspects, and as further described above, the plurality of electrode arrays 104 can comprise a fifth electrode array 104e disposed on the fifth portion 128 of the support structure 102 and a sixth electrode array 104f disposed on the sixth portion of the support structure. In the expanded configuration, the fifth electrode array 104e and the sixth electrode array 104f can be aligned in a third direction that is different than the first and second directions. In exemplary aspects, the second direction can be coincident with a third axis 114 that extends between respective centroids 125 of the fifth and sixth electrode arrays 104e, 104f (and extends into and out of the page in FIG. 2). Optionally, in these aspects, it is contemplated that the third axis 114 does not intersect the first and second axes 110, 112. In further aspects, it is contemplated that the third axis 114 only intersects one of the first axis 110 or the second axis 112. In these aspects, it is contemplated that the third axis 114 can be orthogonal (e.g., perpendicular) or within 1 degree, 5 degrees, 10 degrees, or 15 degrees of being orthogonal or perpendicular to said one of the first axis 110 or the second axis 112. In further aspects, it is contemplated that the first, second, and third axes 110, 112, 114 can intersect each other. Optionally, in these aspects, it is contemplated that the third axis 114 can be orthogonal (e.g., perpendicular) or within 1 degree, 5 degrees, 10 degrees, or 15 degrees of being orthogonal or perpendicular to the first axis 110, and it is further contemplated that the third axis 114 can be orthogonal (e.g., perpendicular) or within 1 degree, 5 degrees, 10 degrees, or 15 degrees of being orthogonal or perpendicular to the second axis 112.

In some optional aspects, in the expanded configuration, it is contemplated that the first axis 110, the second axis 112, and the third axis 114 can each pass through a centroid (e.g., three-dimensional center point) of the support structure 102. In further aspects, it is contemplated that at least two (optionally, only two) of the first, second, and third axes 110, 112, 114 can pass through the centroid of the support structure 102. In still further aspects, it is contemplated that at least one (optionally, only one) of the first, second, and third axes 110, 112, 114 can pass through the centroid of the support structure 102. In still further aspects, it is contemplated that none of the first, second, and third axes 110, 112, 114 passes through the centroid of the support structure 102. More generally, it is contemplated that each electrode array 104 of the plurality of electrode arrays can be aligned with another electrode array of the plurality of electrode arrays along an axis that either passes through the centroid of the support structure 102 or has a selected spacing from the centroid of the support structure to achieve a particular distribution or orientation of TTFields. For example, in some exemplary aspects, a plurality of electrode arrays 104 can be concentrated on one side of the support structure (e.g., one hemisphere of an ovoid support structure) to focus TTFields in a particular way.

As used herein to describe portions of the support structure 102, the term "portion" does not require a particular structure. Exemplary "portions" of the support structure can have planar profiles, arcuate profiles, variable profiles, or combinations thereof. For example, as shown in FIG. 2, each portion 120, 122, 124, 126, 128 can correspond to a portion of a spherical or ovoid shape.

The electrode arrays 104 can optionally comprise a patch 140 with the electrodes 106 disposed thereon. The patch can optionally comprise a flexible and resilient material. It is contemplated that the electrical connections with the electrodes can remain intact as the patch expands, contracts, flexes, or otherwise changes shape. In one exemplary embodiment, the patch has electrodes with electrical connections that do not break as the material of the patch expands, contracts, or changes shape. One exemplary method of forming flexible and expandable conductive materials for the patch includes supersonic cluster beam implantation (SCBI) provided by WISE Srl (Milan, Italy). In further aspects, flexible conductors can be formed with ends separated by a winding/undulating/serpentine path and embedded in a flexible material, which can define a portion of or be incorporated into the patch structure. In this way, the conductors can have an expandable length between said ends and can allow for stretching of the flexible material of the patch. In exemplary aspects, one or more of the patches 140 can be provided as a WISE Cortical Strip (WCS) (WISE Srl, Milan, Italy) or similar structure in which electrodes are embedded within a soft, flexible film (for example, a thin film of silicone).

In further aspects, it is contemplated that the electrodes 106 can be disposed directly onto the support structure 102. Although the electrodes are described herein as arranged in a plurality of electrode arrays, no physical association between electrodes of a given electrode array need be made. For example, it is contemplated that the support structure 102 can have a plurality of independently addressable electrodes disposed thereon, and individual electrodes associated with a particular region on the support structure can be understood to be associated with a specific array. In various aspects, each electrode can couple to a respective lead so that each electrode can be selectively and independently polarized.

The electrode arrays can optionally comprise an odd number of electrodes 106. For example, in some aspects, the electrode array can comprise three, five, seven, nine, or more electrodes. In at least one aspect, one or more (optionally, all) of the electrode arrays can comprise a central electrode 106a and a plurality of circumferential electrodes 106b spaced around the central electrode. In various aspects, each electrode array can optionally comprise the same number of electrodes as the other electrode arrays of the implantable apparatus 100. In other aspects, at least one electrode array can have a different number of electrodes than at least one other electrode array of the implantable apparatus 100. In still other aspects, each electrode array of the implantable apparatus 100 can have a different number of electrodes. In further optional aspects, each of the electrode arrays can comprise an even number of electrodes (e.g., 2, 4, 6, 8, or more electrodes).

### Using the Implantable Apparatus

The implantable apparatus 100, as disclosed herein, can be used to treat tumors in various tissues. In some aspects, for example, the implantable apparatuses 100 can be particularly beneficial in treating brain cancer (e.g., glioblastoma). For example, delivering TTFields to target regions in the brain via trans-dermal arrays can be particularly challenging, primarily due to high resistivity of the skull. Further, TTFields are FDA-approved for treating brain cancer. Accordingly, in some aspects, the target site for providing TTFields can be within the brain of a patient. In further aspects, the target site can be within the torso of a patient. Typically, there is an anatomically well-defined mass comprising contiguous cancer cells, or a shell of cancer cells, surrounding a 'necrotic' region wherein the cells have died due to being starved of nutrients. Often, such a tumor is surgically removed ('resected') and filled with cerebrospinal fluid, which is electrically-conductive and significantly affects an electric field imposed on the region. Typically, a tumor or resection cavity is surrounded by an anatomically undefined or loosely-defined region containing stray cancer cells, since the extent of stray cancer cells in the vicinity of the tumor is dependent on the tumor cell type, and the highly individualized history, anatomy, immune system, etc. of each patient. This region containing stray, non-contiguous cancer cells is referred to herein as a "peritumoral region". Optionally, the target site can comprise a tumor and, in some aspects, a peritumoral region surrounding the tumor. In further aspects, the solid portion of the tumor can be resected as much as possible dependent on surgical access, avoiding damaging tissue, etc., and the peritumoral region can be the target site. In cases in which an inner portion (e.g., a necrotic core) of a tumor is resected and an outer portion of tumor remains, the target site can comprise the outer portion of tumor. In further aspects in which an entirety of the tumor is resected, the peritumoral region can be the target site.

It is contemplated that change in direction of TTFields can advantageously increase efficacy of treatment. In some aspects, TTFields can be provided in at least two orthogonal directions (or, optionally, three orthogonal directions, or multiple directions that can be orthogonal or skew), thereby sweeping the maximum field strength throughout the tumor and peritumoral target region relative to electrically reactive cellular membranes and molecules. Examples of cellular structures and properties affected by electric fields, and which in some cases, magnify or concentrate the field strength thereby increasing its tumor-killing efficacy, are: contact points between contiguous cells, the shape of the cell (notably, ellipsoid vs. spherical, since ellipsoid shape produces inhomogeneous electric fields, which therefore are concentrated in some regions), the thickness of the cell membrane, the thickness of the inner and outer mitochondrial cell membranes and the separation between them, the time constant and electrically-reactive components of cell or mitochondrial membrane ion channels, the cellular furrow during mitosis, the orientation of microtubules, actin filaments, and septin polymers during mitosis, the orientation of other dielectrophoretic, polarizable cellular molecules or organelles, and the orientation of polarizable nanoparticles introduced into the tissue or cell. Examples of sub-cellular structures affected by electric field direction are microtubules, which conduct electric current maximally when aligned with the field, septin (which at different points in the cell cycle is either aligned with or orthogonal to the cell axis), various organelles whose shape and properties make them dielectric (and therefore susceptible to being driven into the cell furrow during mitosis, causing cell blebbing or other deleterious effects), and tubulin dimers, whose orientation relative to the 'plus' end of microtubules during polymerization can be altered by the field and thus polymerization is stalled. Optionally, the TTFields can be generated cyclically, optionally, changing the direction for each cycle. In some aspects, the change of direction of TTFields can be provided at at least 2 Hz, at least 15 Hz, at least 20 Hz, or at least 50 Hz (e.g., from about 15 Hz to about 50 Hz, from about 20 Hz to about 50 Hz, or from about 2 Hz to about 50 Hz), as described in U.S. Patent No. 7,917,227 to Yoram Palti, which is incorporated herein by reference in its entirety. It is contemplated that the embodiments disclosed herein can advantageously be able to change the direction of electric field generated through target areas in both a rapid and accurate manner. For example, the configuration of electrodes can provide flexibility in generating the electric fields as a clinician desires. Accordingly, the electric fields can be configured to treat tumor cells that are positioned in various (e.g., random) orientations.

In some aspects, an implantable apparatus 100 can be inserted into a location in a patient, which location is proximate a target site. For example, in some optional aspects, the implantable apparatus 100 can be positioned so that the support structure 102 is spaced from 0.5-10 cm from a target site, such as from 0.5-5 cm, or from 0.5-3 cm, or from 1-10 cm, or from 1-5 cm, or from 1-3 cm from a target site. In further aspects, the implantable apparatus 100 can be positioned within a peritumoral region (e.g., within a tumor resection cavity), and the peritumoral region can be the target site. Accordingly, in some aspects, at least a portion of a tumor can be resected (e.g., optionally, a necrotic core of the tumor or an entirety of the tumor can be removed), leaving a resection cavity, and the implantable apparatus 100 can then be positioned within the resection cavity. It is contemplated that implanting the implantable apparatus 100 in the resection cavity from a necrotic core resection can preserve a maximum amount of healthy brain tissue affected by a resection procedure. The implantable apparatus 100 can then be used to generate TTFields through the target site.

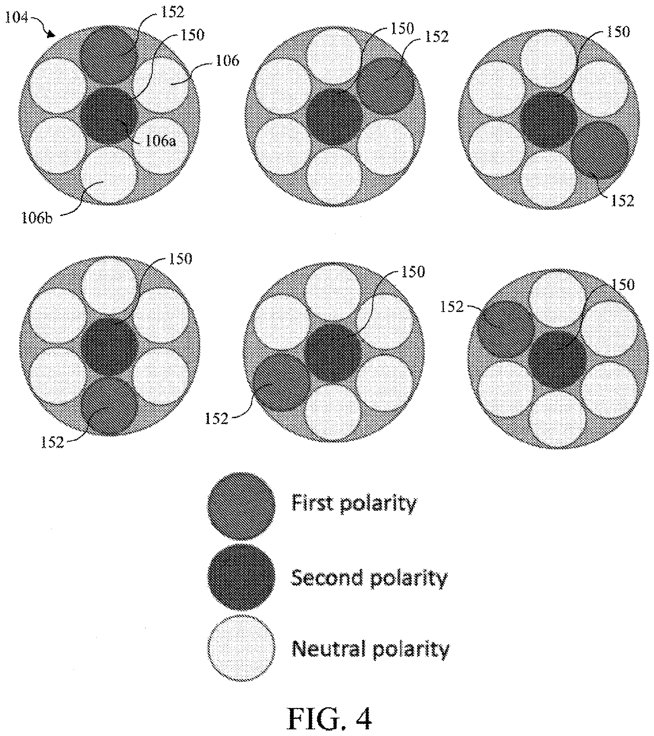

In some aspects, TTFields can be generated between at least two electrodes of the same electrode array 104. Optionally, referring to FIG. 4, the central electrode 106a can provide a first polarity 150 and the circumferential electrodes 106b can sequentially provide a second, opposite polarity 152. For example, in at least one aspect, moving circumferentially around the central electrode, an electric potential can be sequentially provided between the central electrode and each adjacent circumferential electrode. In another aspect, an electric potential can be sequentially provided between the central electrode and non-adjacent circumferential electrodes. Accordingly, it is contemplated that the electrodes 106 can be polarized in the sequence shown in FIG. 4 or out of sequence with the sequence shown in FIG. 4. If the target location is precisely known (e.g. an MRI of a new secondary tumor that has grown from stray cancer cells), the activation of the central electrode and a subset of circumferential electrodes may optimally direct the field toward the tumor and change direction of the field relative to the tumor, given a position of the array. If the target location is not well known, a sequential activation or random activation of the circumferential electrodes in conjunction with the central electrode may optimally 'sweep' the electric field through the peritumoral region and change field direction relative to cells within the peritumoral region.

Further, the precise nature, composition, and geometry of tissue types (e.g. gray matter, white matter, cerebrospinal-filled ventricles, and the tumor cells themselves, etc.) in the target region may not be known. In such cases, purely random activation of electrode patterns may produce optimal field strength throughout the target region.

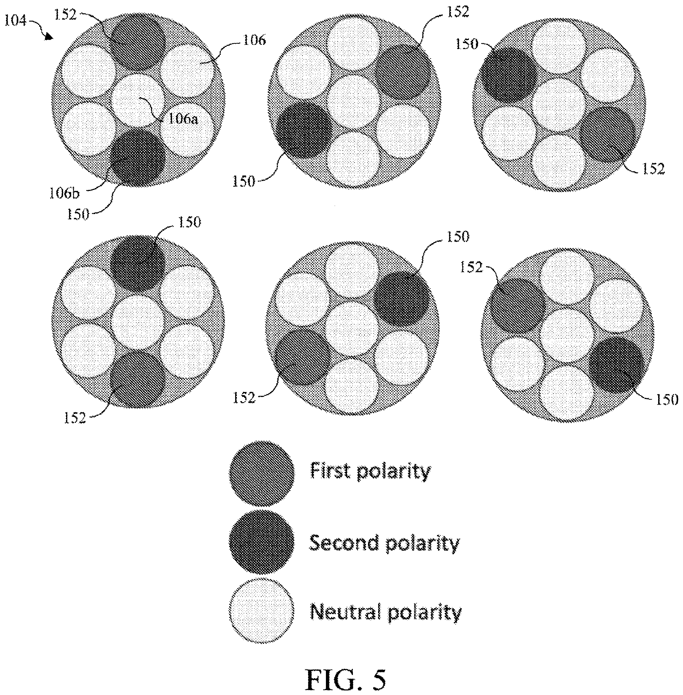

In further aspects, referring to FIG. 5, an electric potential can be sequentially provided between pairs of circumferential electrodes 106b on opposing sides of the central electrode 106a. In some aspects, the sequential pairs of circumferentially spaced electrodes comprise anodes and cathodes that are adjacent to the anodes and cathodes of each pair of electrodes that are polarized immediately prior in the sequence, as shown in sequence in FIG. 5. In further aspects, sequential pairs of circumferentially spaced electrodes comprise anodes and cathodes that are non-adjacent to the anodes and cathodes of each pair of electrodes that are polarized immediately prior in the sequence. Accordingly, it is contemplated that the electrodes 106 can be polarized in the sequence shown in FIG. 5 or out of sequence with the sequence shown in FIG. 5.

In further aspects, TTFields can be generated through the target site by generating electric fields between at least two electrodes of two different electrode arrays of the plurality of electrode arrays. Accordingly, two different electrode arrays can function as a first stimulating electrode array and a second stimulating electrode array. In various aspects, the first and second stimulating electrode arrays can be adjacent (e.g., positioned on adjoining or proximate portions of the support structure 102). In further aspects, the first and second stimulating electrode arrays can be on opposing sides or portions of the support structure 102. It is further contemplated that activation of the first and second stimulating electrode arrays can be alternated in order to generate the TTFields in varying directions. For example, referring to FIG. 2, for a first period, the first and second stimulating electrode arrays can first be the first electrode array 104a and the second electrode array 104b, respectively. Then, for a second period, the first and second stimulating electrode arrays can be the third electrode array 104c and the fourth electrode array 104d, respectively. In this way, an electric field can be generated in two orthogonal directions, thereby beneficially providing direction changes and sweeping the field strength throughout the target region. Optionally, subsequently, for a third period, the first and second stimulating electrode arrays can be the fifth electrode array 104e and the sixth electrode array 104f, respectively. Accordingly, in some aspects, an electric field can be generated in three orthogonal directions, thereby beneficially providing direction changes. Optionally, the polarities can then be reversed to provide the electric field in the opposing directions. For example, for a fourth period, the first and second stimulating electrode arrays can first be the first electrode array 104a and the second electrode array 104b, respectively, and the polarity can be in the opposite direction to the polarity provided during the first period. For a fifth period, the first and second stimulating electrode arrays can first be the third electrode array 104c and the fourth electrode array 104d, respectively, and the polarity can be in the opposite direction to the polarity provided during the second period. For a sixth period, the first and second stimulating electrode arrays can first be the fifth electrode array 104e and the sixth electrode array 104f, respectively, and the polarity can be in the opposite direction to the polarity provided during the third period.

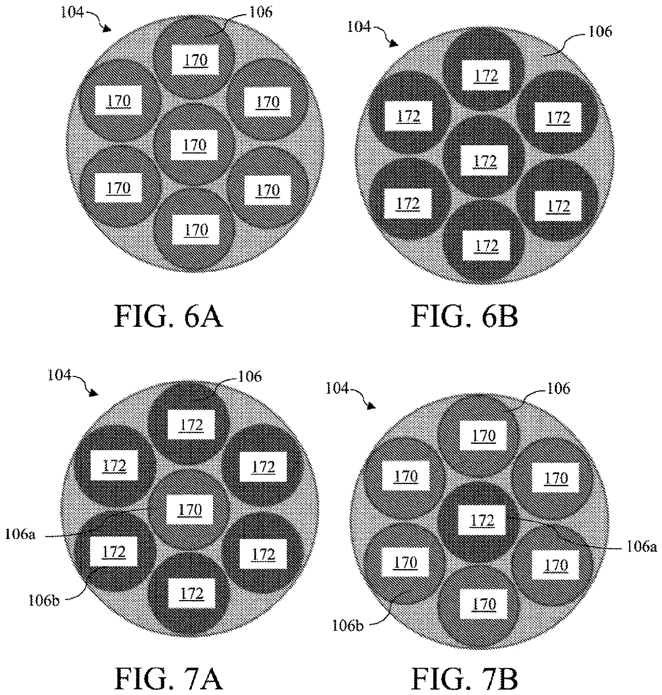

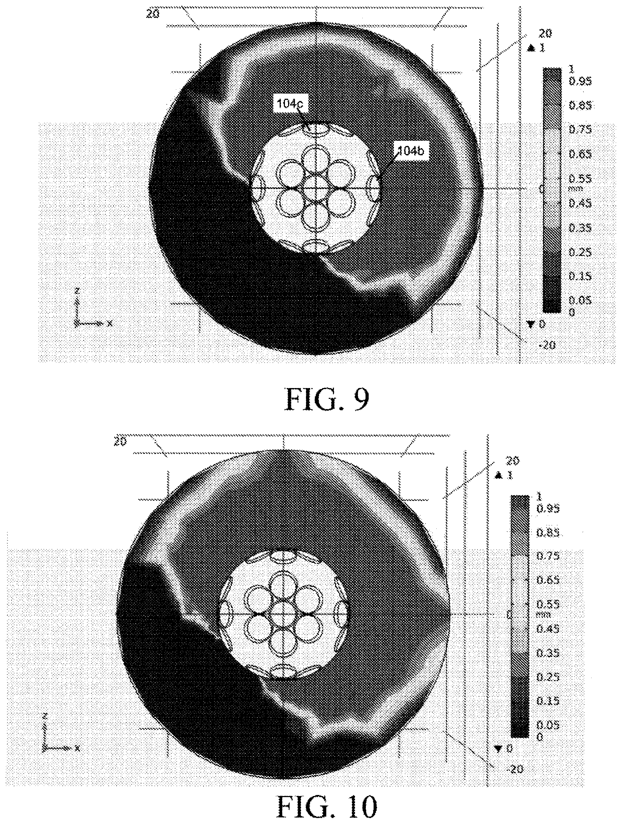

Optionally, referring to FIGS. 6A and 6B, all of the electrodes of the first stimulating electrode array (FIG. 6A) are one of either a cathode or an anode (i.e., provide a first polarity 170), and all of the electrodes of the second stimulating electrode array (FIG. 6B) are the other of the cathode or the anode (i.e., provide a second, opposing polarity 172). It is contemplated that this configuration can generate a maximum field strength between said first and second stimulating electrode arrays over a broader region than an activation pattern effected within the array of a single patch alone, (e.g., a single patch that is configured as is shown in FIGS. 9-11).

In further optional aspects, referring to FIG. 7A and 7B, for each of the first stimulating electrode array and the second stimulating electrode array, the central electrode 106a is an opposite polarity to that of each of the circumferential electrodes 106b. Thus, the first stimulating array (FIG. 7A) can have a first polarity 170 at the central electrode 106a, and the second polarity 172 at each circumferential electrode 106b, and the second stimulating array (FIG. 7B) can have the second polarity 172 at the central electrode 106a, and the first polarity 170 at each circumferential electrode 106b. It is contemplated that such a configuration, e.g., referred to as a "heavily guarded" anode or cathode, can provide relatively deeper penetration depths by forcing current flow into the tissue and therefore a stronger, more efficacious field as compared to all of the electrodes of each array providing the same polarity. In some embodiments, a second array, aligned to face the first array, may have exactly opposite polarities to that of the first array. In further aspects, it is contemplated that the use of a guarded anode or guarded cathode configuration can more reliably focus the electric field in a particular direction. With this additional certainty in field location, it is contemplated that concurrently or sequentially conducted medical imaging procedures (e.g., MRI) can be focused on the particular area where the electric field will be (or has been), thereby improving the effectiveness and usefulness of such medical imaging.

In further optional aspects, referring to FIGS. 8A-8D, the electrodes of each of the first and second stimulating electrode arrays can be polarized in various patterns. FIGS. 8A-8D illustrate different polarization patterns that can be provided for either of the first or second stimulating electrode arrays. For example, any number (e.g., one, two, three, four, or more) electrodes can have the first polarity 170, and some, all, or none of the remaining electrodes can be polarized with the opposite second polarity 172. It should be understood that the polarization patterns of the first and second electrode arrays need not be opposite each other (e.g., with the same pattern but with inverted polarities), although they may. Implantable apparatuses as disclosed herein can provide virtually unlimited combinations of electrode activation to thereby maximize field direction changes through targets. Optionally, each electrode of each of the first stimulating electrode array and the second stimulating electrode array can be randomly selected to be cathodes or anodes. In further optional aspects, each electrode of each of the first stimulating electrode array and the second stimulating electrode array are randomly selected to be cathodes, anodes, or uncharged. In use, it is contemplated that random variations in the polarization patterns of the electrode arrays can help provide better, more uniform coverage of a target area than a set pattern can provide.

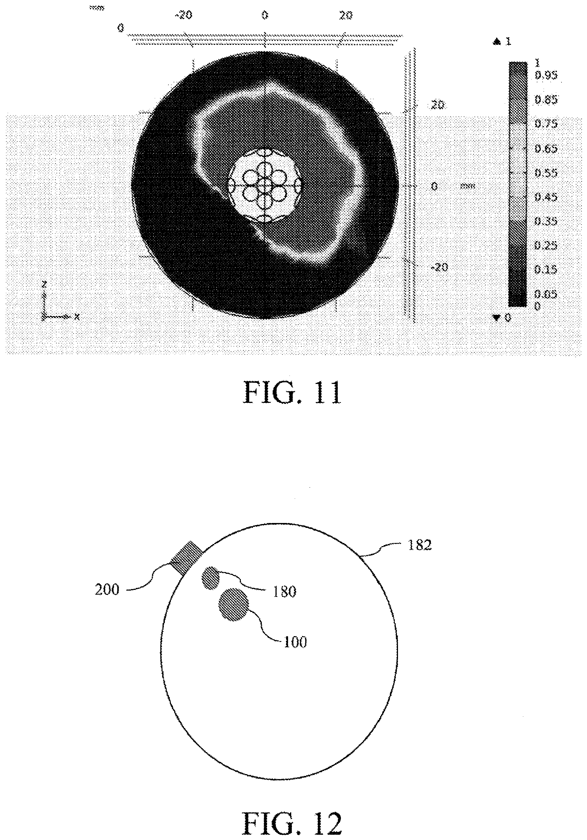

FIGS. 9, 10, and 11 were generated by a computational finite element model of TTFields applied to a simulated tumor/peritumoral region. The figures are two-dimensional slice plots through the x-z axis of a three-dimensional model. The tumor-resection cavity containing the balloon electrode array at its periphery is the clear circular area in the center, and the peritumoral region is the colored area around it. The plot shows a static snapshot of the electric field strength within the peritumoral region generated by an entire patch array (electrode array positioned at portion 104c of the support structure, FIG. 2) activated as a cathode at the "12 o'clock" position (i.e., the top position) on the plot and an entire patch (electrode array positioned at portion 104b of the support structure, FIG. 2) activated as an anode at the "3 o'clock" position (i.e., the right position) on the plot. That is, all of the electrodes 106 (FIG. 1) of the electrode array 104c are provided with a negative charge, and all of the electrodes 106 of the electrode array 104b are provided with a positive charge. The red area and immediately adjacent colors indicated efficacious, tumor-killing field strength. The legend bar shows percent of efficacious field strength (legend bar number x 100%). FIGS. 9 & 10 show the same-sized peritumoral region, having a radius of 20 centimeters, but with more electric current applied to the electrodes in FIG. 10 vs. FIG. 9. FIG. 11 shows a larger peritumoral region, having a radius of 25 centimeters, with the same electric current at the electrodes as in FIG. 9. It can be seen that the patch array design permits covering of a given peritumoral region with efficacious electric field strength by increasing the electric current applied to the electrodes (FIG. 10 compared to FIG. 9), however, the magnitude of the current may be limited by safety considerations such as current density at the electrodes, which can cause chemical reactions or heating effects sufficient to inadvertently damage healthy tissue. These potentially damaging effects typically can be predicted by numerical modeling of the field applied to various tissue compositions and geometries. FIG. 11 shows that the electric field strength does not automatically extend to the outer edge of the peritumoral region, but is distance dependent.

In yet further aspects, it is contemplated that the implantable device 100 can be configured to thermally kill tumor cells by deliberately exceeding damage limits. In this way, thermal ablation and TTFields can be provided via a single apparatus, minimizing damage from requiring implantation of additional equipment. Optionally, the TTFields can be provided simultaneously with thermal ablation. In still further aspects, the implantable device can comprise an outlet that is configured to deliver a chemotherapy agent. Optionally, the device can be used to simultaneously provide chemotherapy and TTFields. The balloon material can enable the chemotherapy agent to perfuse the peritumoral region at a controlled rate while being replenishable. In some aspects, the TTFields can be used to modulate the perfusion rate.

In use, if the target is known to be in between two patch arrays, TTFields can be applied focally in the target region. More typically, the target will be less well-defined, e.g. stray cancer cells, and the field will be swept in orthogonal directions throughout the entire peritumoral region. That protocol can be envisioned by rotating the plot in a circle around the y-axis (note the axes in the lower left corner of the plots in FIGS. 9-11), e.g. in a clockwise fashion, then around the x-axis, and then around the z-axis, thus covering a large portion of the peritumoral region with efficacious field strength and from different directions. It is further contemplated that variations of the patch electrode array patterns can be useful to shape the field for defined targets but can be hard to predict without numerical modeling as is known in the art.

Thus, it is contemplated that generating a first polarity with all of the electrodes of a first array and a second, opposite polarity for all of the electrodes of a second array can be desirable to maximize the area of field coverage, which can be beneficial when precise locations of tumor cells are not known. It is further contemplated that generating electric fields between electrodes on the same array can be advantageous when the target site is precisely known. For example, referring to FIGS. 4 and 5, generating a first polarity at a central electrode and a second, opposite polarity at the surrounding electrodes can be desirable when the exact target area is known.

Optionally, referring to FIG. 12, the implantable apparatus 100 can be a first TTField stimulating apparatus, and a second TTField stimulating apparatus can be positioned so that the target site 180 is disposed between the first and second TTField stimulating apparatuses. For example, the second TTField stimulating apparatus can comprise a transcranial stimulation apparatus 200 that can be positioned against the skull 182 of the patient. TTFields can then be generated between the first TTField stimulating apparatus and the second TTField stimulating apparatus. Multiple transcranial sites can permit changes of direction of the field seen at the target region. More generally, in various aspects, it is contemplated that the second TTField stimulating apparatus can be at least partially (optionally, entirely) positioned on an exterior of the patient's body.

Although the foregoing invention has been described in some detail by way of illustration and example for purposes of clarity of understanding, certain changes and modifications may be practiced within the scope of the appended claims.

## Claims

1. An implantable apparatus for providing tumor treating fields, TTFields, to a target site of a subject, the apparatus comprising:
a support structure (102); and
at least one electrode array (104; 104a-f) disposed on the support structure (102),
**characterized in that**
the at least one electrode array (104; 104a-f) comprises a central electrode (106a) and a plurality of circumferential electrodes (106b) spaced around the central electrode (106a), with the central electrode (106a) and one or more of the
circumferential electrodes (106b) being configured to be activated in sequence or randomly by an
electric potential to provide tumor treating fields of changing orientation through the target site of the subject.

2. The apparatus of claim 1, wherein the central electrode (106a) and one or more of
the circumferential electrodes (106b) are configured to be activated in sequence.

3. The apparatus of claim 1, wherein the central electrode (106a) and one or more of
the circumferential electrodes (106b) are configured to be activated randomly.

4. The apparatus of claim 1, wherein the central electrode (106a) and non-adjacent ones of the circumferential electrodes (106b), optionally to opposite sides of the central electrode (106a), are configured to be activated in sequence.

5. The apparatus of claim 1, wherein the central electrode (106a) and non-adjacent ones of the circumferential electrodes (106b), optionally to opposite sides of the central electrode (106a), are configured to be activated randomly.

6. The apparatus of claim 1, wherein the central electrode (106a) is configured to provide a first polarity (150) and the circumferential electrodes (106b) are configured to provide a second, opposite polarity (152).

7. The apparatus of claim 6, wherein the circumferential electrodes (106b) are configured to be polarized (i) in sequence, (ii) out of sequence, (iii) in subsets or (iv) randomly, optionally sequentially in pairs as anodes and cathodes.

8. The apparatus of claim 1, comprising a plurality of electrode arrays (104; 104a-f).

9. The apparatus of claim 8, wherein the central electrode (106a) of a first electrode array (104; 104a-f) is configured to provide a first polarity (170), the circumferential electrodes (106b) of the first electrode array (104; 104a-f) are configured to provide a second, opposite polarity (172), the central electrode (106a) of the second electrode array (104; 104a-f) is configured to provide the second polarity (172), and the circumferential electrodes (106b) of the second electrode array (104; 104a-f) are configured to provide the first polarity (170).

10. The apparatus of claim 8, wherein any number of the electrodes (106; 106a, b) of each of the electrode arrays (104; 104a-f) are configured to provide a first polarity (170), and some, all or none of the remaining electrodes (106; 106a, b) of the electrode arrays (104; 104a-f) are configured to provide a second, opposite polarity (172).

11. The apparatus of claim 8, wherein the electrode arrays (104; 104a-f) comprise the same number or a different number of electrodes (106; 106a, b), optionally each of the electrode arrays (104; 104a-f) comprises a different number of electrodes (106; 106a, b).

12. The apparatus of claim 1, comprising first and second electrode arrays (104a, b) disposed on first and second portions (120, 122) on opposing sides of the support structure (102), optionally the first and second electrode arrays (104a, b) are aligned in a first direction, optionally co-incident with a first axis that extends between the centroids of the first and second electrode arrays (104a, b).

13. The apparatus of claim 1, comprising first, second, third and fourth electrode arrays (104a-d) disposed on first, second, third and fourth portions (120, 122, 124, 126) on opposing sides of the support structure (102), optionally the first and second electrode arrays (104a, b) are aligned in a first direction, optionally co-incident with a first axis that extends between the centroids of the first and second electrode arrays (104a, b), optionally the third and fourth electrode arrays (104c, d) are aligned in a second direction different to the first direction, optionally co-incident with a second axis that extends between the centroids of the third and fourth electrode arrays (104c, d), optionally the first and second axes intersect or do not intersect, optionally the second axis is within 15 degrees of being orthogonal to the first axis.

14. The apparatus of claim 1, comprising first, second, third, fourth, fifth and sixth electrode arrays (104a-f) disposed on first, second, third, fourth, fifth and sixth portions (120, 122, 124, 126, 128) on opposing sides of the support structure (102), optionally the first and second electrode arrays (104a, b) are aligned in a first direction, optionally co-incident with a first axis that extends between the centroids of the first and second electrode arrays (104a, b), optionally the third and fourth electrode arrays (104c, d) are aligned in a second direction different to the first direction, optionally co-incident with a second axis that extends between the centroids of the third and fourth electrode arrays (104c, d), optionally the first and second axes intersect or do not intersect, optionally the second axis is within 15 degrees of being orthogonal to the first axis, optionally the fifth and sixth electrode arrays (104e, f) are aligned in a third direction different to the first and second directions, optionally co-incident with a third axis that extends between the centroids of the fifth and sixth electrode arrays (104e, f), optionally the third axis intersects only one of the first and second axes or neither of the first and second axes, optionally the third axis is within 15 degrees of being orthogonal to the first axis, optionally the third axis is within 15 degrees of being orthogonal to the second axis.

## Patentansprüche

1. Implantierbare Vorrichtung zur Bereitstellung von Tumorbehandlungsfeldern, TTFeldern, an einem Zielort einer Person, worin die Vorrichtung umfasst:
eine Trägerstruktur (102); und
mindestens eine Elektrodenanordnung (104; 104a-f), die auf der Trägerstruktur (102) angeordnet ist,
**dadurch gekennzeichnet, dass** die mindestens eine Elektrodenanordnung (104; 104a-f) eine Zentralelektrode (106a) und mehrere Umfangselektroden (106b) umfasst, die um die Zentralelektrode (106a) herum beabstandet sind, worin die Zentralelektrode (106a) und eine oder mehrere der Umfangselektroden (106b) ausgestaltet sind, nacheinander oder zufällig durch ein elektrisches Potenzial aktiviert zu werden, um Tumorbehandlungsfelder mit wechselnder Ausrichtung durch den Zielort des Patienten bereitzustellen.

2. Vorrichtung nach Anspruch 1, worin die Zentralelektrode (106a) und eine oder mehrere der Umfangselektroden (106b) ausgestaltet sind, nacheinander aktiviert zu werden.

3. Vorrichtung nach Anspruch 1, worin die Zentralelektrode (106a) und eine oder mehrere der Umfangselektroden (106b) ausgestaltet sind, zufällig aktiviert zu werden.

4. Vorrichtung nach Anspruch 1, worin die Zentralelektrode (106a) und nicht benachbarte der Umfangselektroden (106b), die sich wahlweise auf gegenüberliegenden Seiten der zentralen Elektrode (106a) befinden, ausgestaltet sind, nacheinander aktiviert zu werden.

5. Vorrichtung nach Anspruch 1, worin die Zentralelektrode (106a) und nicht benachbarte der Umfangselektroden (106b), die sich wahlweise auf gegenüberliegenden Seiten der Zentralelektrode (106a) befinden, ausgestaltet sind, zufällig aktiviert zu werden.

6. Vorrichtung nach Anspruch 1, worin die Zentralelektrode (106a) ausgestaltet ist, eine erste Polarität (150) zu liefern, und die Umfangselektroden (106b) ausgestaltet sind, eine zweite, entgegengesetzte Polarität (152) zu liefern.

7. Vorrichtung nach Anspruch 6, worin die Umfangselektroden (106b) ausgestaltet sind, (i) nacheinander, (ii) außer der Reihe, (iii) in Untergruppen oder (iv) zufällig, wahlweise nacheinander in Paaren als Anoden und Kathoden, polarisiert zu werden.

8. Vorrichtung nach Anspruch 1, welche mehrere Elektrodenanordnungen (104; 104a-f) umfasst.

9. Vorrichtung nach Anspruch 8, worin die Zentralelektrode (106a) einer ersten Elektrodenanordnung (104; 104a-f) ausgestaltet ist, eine erste Polarität (170) bereitzustellen, die Umfangselektroden (106b) der ersten Elektrodenanordnung (104; 104a-f) ausgestaltet sind, eine zweite, entgegengesetzte Polarität (172) bereitzustellen, die Zentralelektrode (106a) der zweiten Elektrodenanordnung (104; 104a-f) ausgestaltet ist, die zweite Polarität (172) bereitzustellen, und die Umfangselektroden (106b) der zweiten Elektrodenanordnung (104; 104a-f) ausgestaltet sind, die erste Polarität (170) bereitzustellen.

10. Vorrichtung nach Anspruch 8, worin eine beliebige Anzahl der Elektroden (106; 106a, b) jeder der Elektrodenanordnungen (104; 104a-f) ausgestaltet ist, eine erste Polarität (170) bereitzustellen, und einige, alle oder keine der übrigen Elektroden (106; 106a, b) der Elektrodenanordnungen (104; 104a-f) ausgestaltet sind, eine zweite, entgegengesetzte Polarität (172) bereitzustellen.

11. Vorrichtung nach Anspruch 8, worin die Elektrodenanordnungen (104; 104a-f) die gleiche Anzahl oder eine unterschiedliche Anzahl von Elektroden (106; 106a, b) umfassen, wahlweise worin jede der Elektrodenanordnungen (104; 104a-f) eine unterschiedliche Anzahl von Elektroden (106; 106a, b) umfasst.

12. Vorrichtung nach Anspruch 1, welche erste und zweite Elektrodenanordnungen (104a, b) umfasst, die auf ersten und zweiten Abschnitten (120, 122) auf gegenüberliegenden Seiten der Trägerstruktur (102) angeordnet sind, wahlweise worin die ersten und zweiten Elektrodenanordnungen (104a, b) in einer ersten Richtung ausgerichtet sind, die wahlweise mit einer ersten Achse zusammenfällt, die sich zwischen den Mittelpunkten der ersten und zweiten Elektrodenanordnungen (104a, b) erstreckt.

13. Vorrichtung nach Anspruch 1, welche erste, zweite, dritte und vierte Elektrodenanordnungen (104a-d) umfasst, die auf ersten, zweiten, dritten und vierten Abschnitten (120, 122, 124, 126) auf gegenüberliegenden Seiten der Trägerstruktur (102) angeordnet sind, wahlweise worin die ersten und zweiten Elektrodenanordnungen (104a, b) in einer ersten Richtung ausgerichtet sind, die wahlweise mit einer ersten Achse zusammenfällt, die sich zwischen den Mittelpunkten der ersten und zweiten Elektrodenanordnungen (104a, b) erstreckt, wahlweise worin die dritte und vierte Elektrodenanordnung (104c, d) in einer zweiten Richtung ausgerichtet sind, die sich von der ersten Richtung unterscheidet und wahlweise mit einer zweiten Achse zusammenfällt, die sich zwischen den Mittelpunkten der dritten und vierten Elektrodenanordnung (104c, d) erstreckt, wahlweise worin sich die erste und zweite Achse schneiden oder nicht schneiden, wahlweise worin die zweite Achse innerhalb von 15 Grad orthogonal zur ersten Achse ist.

14. Vorrichtung nach Anspruch 1, welche erste, zweite, dritte, vierte, fünfte und sechste Elektrodenanordnungen (104a-f) umfasst, die auf ersten, zweiten, dritten, vierten, fünften und sechsten Abschnitten (120, 122, 124, 126, 128) auf gegenüberliegenden Seiten der Trägerstruktur (102) angeordnet sind, wahlweise worin die ersten und zweiten Elektrodenanordnungen (104a, b) in einer ersten Richtung ausgerichtet sind, die wahlweise mit einer ersten Achse zusammenfällt, die sich zwischen den Mittelpunkten der ersten und zweiten Elektrodenanordnungen (104a, b) erstreckt, wahlweise worin die dritte und vierte Elektrodenanordnung (104c, d) in einer zweiten Richtung ausgerichtet sind, die sich von der ersten Richtung unterscheidet und wahlweise mit einer zweiten Achse zusammenfällt, die sich zwischen den Mittelpunkten der dritten und vierten Elektrodenanordnung (104c, d) erstreckt, wahlweise worin sich die erste und zweite Achse schneiden oder nicht schneiden, wahlweise die zweite Achse innerhalb von 15 Grad orthogonal zur ersten Achse ist, wahlweise worin die fünfte und sechste Elektrodenanordnung (104e. i) in einer dritten Richtung ausgerichtet sind, die sich von der ersten und der zweiten Richtung unterscheidet und wahlweise mit einer dritten Achse zusammenfällt, die sich zwischen den Mittelpunkten der fünften und der sechsten Elektrodenanordnung (104e, f) erstreckt, wahlweise worin die dritte Achse nur eine der ersten und der zweiten Achse oder keine der ersten und der zweiten Achse schneidet, wahlweise worin die dritte Achse innerhalb von 15 Grad orthogonal zur ersten Achse ist, wahlweise worin die dritte Achse innerhalb von 15 Grad orthogonal zur zweiten Achse ist.

## Revendications

1. - Appareil implantable pour fournir des champs de traitement de tumeur, ChampsTT, à un site cible d'un sujet, l'appareil comprenant:
une structure de support (102); et
au moins un réseau d'électrodes (104 ; 104a-f) disposé sur la structure de support (102),
**caractérisé par le fait que** l'au moins un réseau d'électrodes (104 ; 104a-f) comprend une électrode centrale (106a) et une pluralité d'électrodes circonférentielles (106b) espacées autour de l'électrode centrale (106a), l'électrode centrale (106a) et une ou plusieurs des électrodes circonférentielles (106b) étant configurées pour être activées en séquence ou de manière aléatoire par un potentiel électrique pour fournir des champs de traitement de tumeur d'orientation changeante à travers le site cible du sujet.

2. - Appareil selon la revendication 1, dans lequel l'électrode centrale (106a) et une ou plusieurs des électrodes circonférentielles (106b) sont configurées pour être activées en séquence.

3. - Appareil selon la revendication 1, dans lequel l'électrode centrale (106a) et une ou plusieurs des électrodes circonférentielles (106b) sont configurées pour être activées de manière aléatoire.

4. - Appareil selon la revendication 1, dans lequel l'électrode centrale (106a) et des électrodes non adjacentes parmi les électrodes circonférentielles (106b), facultativement sur des côtés opposés de l'électrode centrale (106a), sont configurées pour être activées en séquence.

5. - Appareil selon la revendication 1, dans lequel l'électrode centrale (106a) et des électrodes non adjacentes parmi les électrodes circonférentielles (106b), facultativement sur des côtés opposés de l'électrode centrale (106a), sont configurées pour être activées de manière aléatoire.

6. - Appareil selon la revendication 1, dans lequel l'électrode centrale (106a) est configurée pour fournir une première polarité (150) et les électrodes circonférentielles (106b) sont configurées pour fournir une seconde polarité opposée (152).

7. - Appareil selon la revendication 6, dans lequel les électrodes circonférentielles (106b) sont configurées pour être polarisées (i) en séquence, (ii) hors séquence, (iii) en sous-ensembles ou (iv) de manière aléatoire, facultativement séquentiellement en paires en tant qu'anodes et cathodes.

8. - Appareil selon la revendication 1, comprenant une pluralité de réseaux d'électrodes (104 ; 104a-f).

9. - Appareil selon la revendication 8, dans lequel l'électrode centrale (106a) d'un premier réseau d'électrodes (104 ; 104a-f) est configurée pour fournir une première polarité (170), les électrodes circonférentielles (106b) du premier réseau d'électrodes (104 ; 104a-f) sont configurées pour fournir une seconde polarité opposée (172), l'électrode centrale (106a) du deuxième réseau d'électrodes (104 ; 104a-f) est configurée pour fournir la seconde polarité (172), et les électrodes circonférentielles (106b) du deuxième réseau d'électrodes (104 ; 104a-f) sont configurées pour fournir la première polarité (170).

10. - Appareil selon la revendication 8, dans lequel un nombre quelconque des électrodes (106 ; 106a, b) de chacun des réseaux d'électrodes (104 ; 104a-f) sont configurées pour fournir une première polarité (170), et certaines, toutes ou aucune des électrodes restantes (106 ; 106a, b) des réseaux d'électrodes (104 ; 104a-f) sont configurées pour fournir une seconde polarité opposée (172).

11. - Appareil selon la revendication 8, dans lequel les réseaux d'électrodes (104 ; 104a-f) comprennent le même nombre ou un nombre différent d'électrodes (106 ; 106a, b), facultativement chacun des réseaux d'électrodes (104 ; 104a-f) comprend un nombre différent d'électrodes (106 ; 106a, b) .

12. - Appareil selon la revendication 1, comprenant des premier et deuxième réseaux d'électrodes (104a, b) disposés sur des première et deuxième parties (120, 122) sur des côtés opposés de la structure de support (102), facultativement les premier et deuxième réseaux d'électrodes (104a, b) sont alignés dans une première direction, facultativement coïncidente avec un premier axe qui s'étend entre les centroïdes des premier et deuxième réseaux d'électrodes (104a, b).

13. - Appareil selon la revendication 1, comprenant des premier, deuxième, troisième et quatrième réseaux d'électrodes (104a-d) disposés sur des première, deuxième, troisième et quatrième parties (120, 122, 124, 126) sur des côtés opposés de la structure de support (102), facultativement les premier et deuxième réseaux d'électrodes (104a, b) sont alignés dans une première direction, facultativement coïncidente avec un premier axe qui s'étend entre les centroïdes des premier et deuxième réseaux d'électrodes (104a, b), facultativement les troisième et quatrième réseaux d'électrodes (104c, d) sont alignés dans une deuxième direction différente de la première direction, facultativement coïncidente avec un deuxième axe qui s'étend entre les centroïdes des troisième et quatrième réseaux d'électrodes (104c, d), facultativement les premier et deuxième axes se croisent ou ne se croisent pas, facultativement le deuxième axe est orthogonal au premier axe à 15 degrés près.

14. - Appareil selon la revendication 1, comprenant des premier, deuxième, troisième, quatrième, cinquième et sixième réseaux d'électrodes (104a-f) disposés sur des première, deuxième, troisième, quatrième, cinquième et sixième parties (120, 122, 124, 126, 128) sur des côtés opposés de la structure de support (102), facultativement les premier et deuxième réseaux d'électrodes (104a, b) sont alignés dans une première direction, facultativement coïncidente avec un premier axe qui s'étend entre les centroïdes des premier et deuxième réseaux d'électrodes (104a, b), facultativement les troisième et quatrième réseaux d'électrodes (104c, d) sont alignés dans une deuxième direction différente de la première direction, facultativement coïncidente avec un deuxième axe qui s'étend entre les centroïdes des troisième et quatrième réseaux d'électrodes (104c, d), facultativement les premier et deuxième axes se croisent ou ne se croisent pas, facultativement le deuxième axe est orthogonal au premier axe à 15 degrés près, facultativement les cinquième et sixième réseaux d'électrodes (104e, f) sont alignés dans une troisième direction différente des première et deuxième directions, facultativement coïncidente avec un troisième axe qui s'étend entre les centroïdes des cinquième et sixième réseaux d'électrodes (104e, f), facultativement le troisième axe ne coupe qu'un seul des premier et deuxième axes ou aucun des premier et deuxième axes, facultativement le troisième axe est orthogonal au premier axe à 15 degrés près, facultativement le troisième axe est orthogonal au deuxième axe à 15 degrés près.
